# EUROPEAN PATENT SPECIFICATION

(11) **EP 1 849 419 B1**
(45) Date of publication and mention of the grant of the patent: **07.09.2016**
(21) Application number: 06714004.6
(22) Date of filing: 17.02.2006
(51) Int. Cl.: A61B 17/221, A61B 1/00, A61B 18/14

(54) **TREATMENT INSTRUMENT FOR ENDOSCOPE, AND ENDOSCOPE SYSTEM**
BEHANDLUNGSVORRICHTUNG FÜR ENDOSKOPE UND ENDOSKOPSYSTEM
INSTRUMENT DE TRAITEMENT POUR UN ENDOSCOPE ET SYSTÈME ENDOSCOPIQUE

(30) Priority: 18.02.2005 JP 2005042241
(43) Date of publication of application: 31.10.2007
(73) Proprietor: Olympus Corporation, Tokyo 192-8507 (JP)
(72) Inventor: OKADA, Tsutomu, 2951 Ishikawa-machi, Hachioji-shi, Tokyo 192-8507 (JP)
(74) Representative: von Hellfeld, Axel
(86) International application number: PCT/JP2006/302863
(87) International publication number: WO 2006/088148

(56) References cited:
- JP-A- 05 038 342
- JP-A- 08 336 542
- JP-A- 2002 051 974
- JP-A- 2002 224 136
- JP-A- 2004 321 822
- JP-A- 2004 337 442
- JP-B2- 07 083 749
- US-A- 4 407 273
- US-A- 4 840 176
- US-A- 5 163 938
- US-A- 5 766 184
- US-A1- 2004 077 929

## Description

### Technical Field

The present invention relates to an endoscopic treatment apparatus used in combination with an endoscope, and an endoscope system.

### Background Art

Jpn. Pat. Appln. KOKAI Publication No. 2002-51974, US Pat. Specification No. 5897554 and German UM Specification No. 7715649 disclose an endoscopic treatment apparatus capable of controlling and adjusting a position of a portion to be treated. Such treatment apparatuses for endoscopes are a high-frequency snare or a spatula type knife. The endoscopic treatment apparatus of this type has a loop or spatula-shaped electrode as a treatment unit. Such a loop or spatula-type electrode has a directivity that differs according to the treatment direction. Thus, such a treatment unit must be placed in a specific direction upon use. This requires adjustment to face the treatment unit in a suitable direction to an object part in the body cavity.

The Jpn. Pat. Appln. KOKAI Publication No. 2002-51974 discloses the endoscopic treatment apparatus relating to a high-frequency snare. The treatment apparatus is provided with a rotation control unit. Therefore, a loop-shaped snare wire and an operation wire to operate the snare wire are prevented from rotationally moving with respect to a flexible tube.

The US Pat. Specification No. 5897554 discloses the endoscopic treatment apparatus relating to a catheter provided with a loop electrode for resection. The catheter is capable of deflecting the loop electrode in two ways by operating an operation wire connected to a flat wire provided at a proximal end of the loop electrode.

The German UM Specification, No. 7715649, discloses the endoscopic treatment apparatus relating to a so-called Schlinge loop guide. The guide device is inserted into the stomach through an endoscope channel for the stomach. The treatment apparatus is placed in a loop guide (sheath). A portion close to the distal end of a tensile wire connected to the proximal end of a loop is shaped like a strip. When the loop guide is bent to a polyp through a gastroscope, a portion of the loop guide is shaped flat. The direction of the loop can be adjusted by guiding the strip-shaped portion.

The high-frequency snare disclosed in the Jpn. Pat. Appln. KOKAI Publication No. 2002-51974 controls a direction of a snare wire by defining the sectional shape of the snare wire according to the inside shape of a flexible tube. Thus, when changing the direction of the snare wire with respect to an endoscope in the state that the snare wire is inserted into an endoscope channel, the operator must turn the flexible tube itself to the near-hand side, and this adjustment of direction is troublesome.

The resection catheter disclosed in the US Pat. Specification No. 5897554 is inserted alone into the heart through the skin without passing through an endoscope. Therefore, directing the loop electrode to an object part itself is difficult.

In the Schlinge loop guide disclosed in the German UM Specification No. 7715649 , the flat portion is formed in the sheath itself by bending the sheath itself by bending a gastroscope. The portion shaped like a strip is adjusted to be passed through the inside shape of the flat sheath portion, thereby adjusting the direction of the loop. Therefore, the loop direction cannot be adjusted in the state that an insertion section of a gastroscope cannot be bent. Besides, when adjusting the loop direction, it is necessary to adjust relatively tightly the flat portion of the sheath to the portion shaped like a strip. Therefore, the adjustment of the loop direction is troublesome. This affects endoscopic observation.

US 4 840 176 A discloses a treating instrument comprising a sheath for insertion into a inserting channel of an endoscope. The instrument further comprises a snare wire connected to a first connecting member movably accommodated in the sheath, an elongated plate coupled to the first connecting member, an elongated member, a second connecting member coupled to the plate and the elongated member and an operating handle connected to the proximal end of elongated member. A rotation-restricting pin is attached to the wall of sheath 4 and prevents the rotation of the plate.

JP 2002 051974 A discloses a high frequency snare comprising a hand operating part and an insert portion. The insert portion comprises a sheath, an operation wire inserted in the sheath and a snare wire provided at the distal end of the operation wire.

US 5 163 938 A discloses a high-frequency surgical knife with an operation section and a flexible sheath. A flexible, electrically conductive wire is provided partially inside and partially outside the sheath. A direction control member made of a flexible flat sheet is provided in the distal end portion of the sheath.

US 5 766 184 A discloses a clip device as an endoscopic treatment tool comprising a clip device proper and an operating unit. The clip device proper comprises a flexible lead tube and a flexible operating tube retractively inserted in the lead tube. An operating wire having a torque transmissivity is retractively inserted in the operating tube. A hook mounted to the forward end of the operating wire is adapted to replaceably engage a coupling plate of the cassette-type clip unit.

US 2004/077929 A1 discloses a catheter comprising a flexible tubular insert section having notches, an operating wire, and a reinforcing tube.

US 4 407 273 A discloses an endoscope comprising an optical fibre bundle for illumination communicating with an illumination window, an optical fibre bundle for observation communicating with a viewing window, an opening through which an implement is projected out, and a raiser adapted to bend the forward end of the implement to be guided into the visual field of the viewing window.

### Disclosure of Invention

It is an object of the present invention to provide an endoscopic treatment apparatus and an endoscope system, which are capable of facing a treating portion in a desired direction without causing a serious problem in endoscopic observation, or affecting the bending of an insertion section of an endoscope.

An endoscopic treatment apparatus according to claim 1 is provided.

Furthermore, an endoscope system according to claim 10 is provided.

### Brief Description of Drawings

FIG. 1 is a schematic diagram showing the entire high-frequency snare according to a first embodiment of the invention;
FIG. 2A is a schematic longitudinal sectional view showing a distal end portion of the high-frequency snare according to the first embodiment, taken along line IIA-IIA in FIG. 2B;
FIG. 2B is a schematic longitudinal sectional view showing the distal end portion of the high-frequency snare according to the first embodiment, taken along line IIB-IIB in FIG. 2A;
FIG. 3 is a schematic transverse sectional view of the high-frequency snare according to the first embodiment;
FIG. 4A is a schematic diagram showing the state that the high-frequency snare according to the first embodiment is used in combination with an endoscope;
FIG. 4B is a schematic diagram showing the state that the high-frequency snare according to the first embodiment is used in combination with the endoscope;
FIG. 4C is a modification of a schematic diagram showing the state that the high-frequency snare according to the first embodiment is used in combination with the endoscope;
FIG. 5 is a schematic transverse sectional view of a modification of a flexible tube and a plate member of the high-frequency snare according to the first embodiment;
FIG. 6A is a schematic longitudinal sectional view of the distal end of the high-frequency snare according to a second embodiment, taken along the line VIA-VIA in FIG. 6B;
FIG. 6B is a schematic longitudinal sectional view of the distal end of the high-frequency snare according to the second embodiment, taken along the line VIB-VIB in FIG. 6A;
FIG. 7 is a schematic transverse sectional view of the high-frequency snare according to the second embodiment, taken along the line VII-VII in FIG. 6A;
FIG. 8A is a schematic longitudinal sectional view of the distal end of the high-frequency snare according to a third embodiment, taken along the line VIIIA-VIIIA in FIG. 8B;
FIG. 8B is a schematic longitudinal sectional view of the distal end of the high-frequency snare according to the third embodiment, taken along the line VIIIB-VIIIB in FIG. 8A;
FIG. 9 is a schematic transverse sectional view of the high-frequency snare according to the third embodiment, taken along the line IX-IX in FIG. 8A;
FIG. 10 is a schematic diagram showing the state that the high-frequency snare according to the third embodiment is used in combination with an endoscope;
FIG. 11 is a schematic longitudinal sectional view of the distal end of the high-frequency snare according to a fourth embodiment;
FIG. 12 is a schematic transverse sectional view of the high-frequency snare according to the fourth embodiment, taken along the line XII-XII in FIG. 11;
FIG. 13A is a schematic diagram showing the state that the high-frequency snare according to the fourth embodiment is used in combination with the endoscope;
FIG. 13B is a schematic diagram showing the state that the high-frequency snare according to the fourth embodiment is used in combination with the endoscope;
FIG. 14 is a schematic diagram showing the entire high-frequency snare according to a fifth embodiment of the invention;
FIG. 15A is a schematic longitudinal sectional view of the distal end of the high-frequency snare according to the fifth embodiment, taken along the line XVA-XVA in FIG. 15B;
FIG. 15B is a schematic longitudinal sectional view of the distal end of the high-frequency snare according to the fifth embodiment, taken along the line XVB-XVB in FIG. 15A;
FIG. 16 is a schematic diagram showing the state that the high-frequency snare according to the fifth embodiment is used in combination with the endoscope;
FIG. 17A is a schematic longitudinal sectional view of the distal end of the high-frequency snare according to a sixth embodiment, taken along the line XVIIA-XVIIA in FIG. 17B;
FIG. 17B is a schematic longitudinal sectional view of the distal end of the high-frequency snare according to the sixth embodiment, taken along the line XVIIB-XVIIB in FIG. 17A;
FIG. 18 is a schematic transverse sectional view of the high-frequency snare according to the sixth embodiment, taken along the line XVIII-XVIII in FIG. 17B;
FIG. 19 is a schematic transverse sectional view of a modification of the high-frequency snare according to the sixth embodiment, taken along the line XVIII-XVIII in FIG. 17B;
FIG. 20 is a schematic diagram showing the entire high-frequency knife according to a seventh embodiment of the invention;
FIG. 21A is a schematic longitudinal sectional view of the distal end of the high-frequency knife according to the seventh embodiment, taken along the line XXIA-XXIA in FIG. 21B;
FIG. 21B is a schematic longitudinal sectional view of the distal end of the high-frequency knife according to the seventh embodiment, taken along the line XXIB-XXIB in FIG. 21A;
FIG. 22A is a schematic diagram showing the state that the high-frequency knife according to the seventh embodiment is used in combination with the endoscope;
FIG. 22B is a schematic diagram showing the state that the high-frequency knife according to the seventh embodiment is used in combination with the endoscope; and
FIG. 23 is a schematic diagram showing the state that a tissue layer under a mucous membrane is cut through use of both the high-frequency knife according to the seventh embodiment and the endoscope.

### Best Mode for Carrying Out the Invention

The best mode for carrying out the invention will be explained hereinafter with reference to the accompanying drawings.

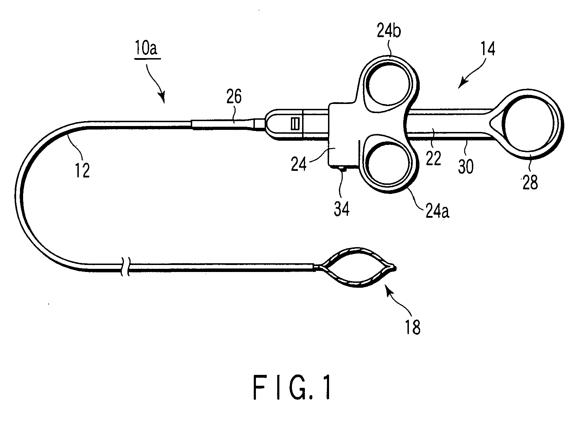

A high-frequency incision/resection apparatus according to a first embodiment of the invention will be explained first with reference to FIGS. 1 to 5.

A high-frequency snare 10a as the high-frequency incision/resection apparatus shown in FIGS. 1 to3 is used in combination with an endoscope 60 in many cases. Namely, the high-frequency snare 10a and endoscope 60 form an endoscope system.

The high-frequency snare 10a includes a flexible tube (flexible sheath) 12, an operation handle 14, a movable body 16, a treatment portion 18. In this embodiment, an incision wire is to be provided in the treatment portion 18.

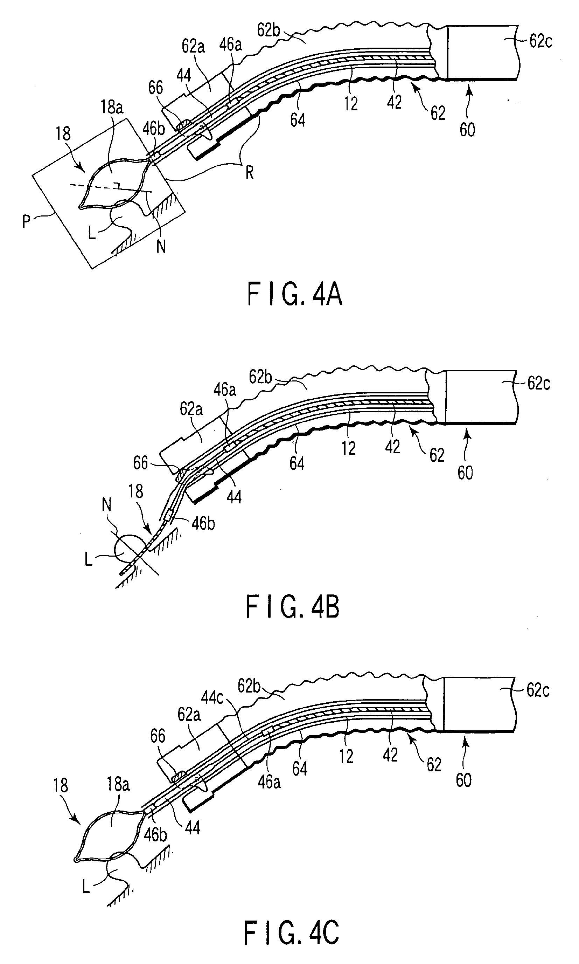

The flexible tube 12 is a non-conductor with flexibility, and shaped like a thin tube. The flexible tube 12 can be inserted into the treatment apparatus insertion channel 64 of an insertion section 62 of the endoscope 60 shown in FIGS. 4A to 4C. The operation handle 14 is provided in the proximal end portion of the flexible tube 12.

The operation handle 14 shown in FIG. 1 is made of a hard non-conductor such as a plastic material. The operation handle 14 has a base member 22 and a slider (movable member) 24.

The base member 22 is formed substantially straight, for example along the axial direction of the flexible tube 12. The proximal end of the flexible tube 12 is connected to the distal end of the base member 22. At the distal end of the base member 22, a protection hood 26 is provided to prevent buckling of the proximal end portion of the flexible tube 12. At the proximal end of the base member 22, a ring 28 to fit such as the thumb of the operator is formed as one body. The base member 22 includes a slider receiving part 30 shaped substantially rectangular with a substantially constant thickness along the axial direction of the flexible tube, between the distal end portion and proximal end portion. In the slider receiving part 30, a slider 24 is provided. The slider 24 is provided with rings 24a and 24b to fit such as the forefinger and middle finger of an operator as one body. The slider 24 is connected to the proximal end of the movable body 16. Therefore, when the slider 24 is slid along the slider receiving part 30 of the base member 22, the movable body 16 is moved along the axial direction inside (inside the lumen) of the flexible tube 12.

Although not shown, the slider 24 is connected to the proximal end portion of the movable body 16. Therefore, the movable body 16 is moved inside the flexible tube 12 when the slider 24 slides the slider receiving part 30 of the base member 22.

The slider 24 is further provided with an electrode 34. The electrode 34 is electrically connected to the movable body 16 inside the slider 24. The electrode 34 is connectable to and removable from a not-shown high-frequency power supply through a not-shown cable.

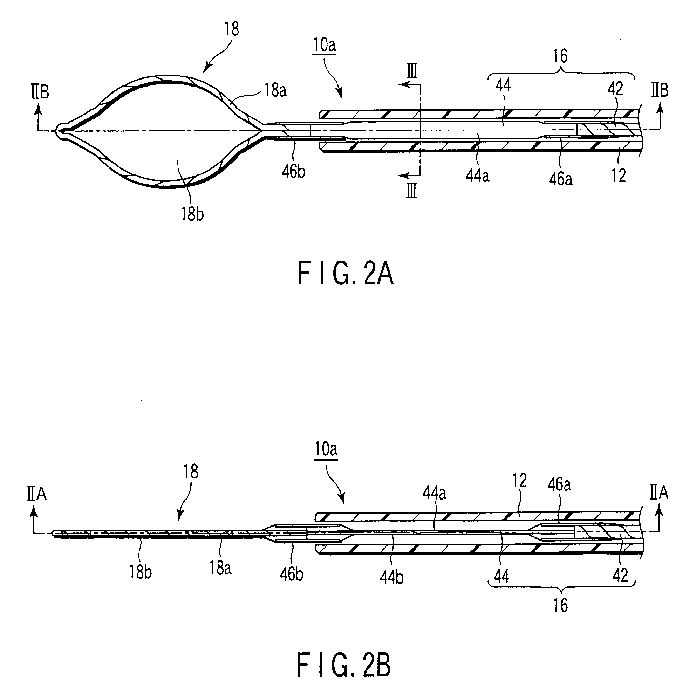

As shown in FIGS. 2A and 2B, the movable body 16 includes an operation wire 42 and a strip-like plate member (direction control member) 44. The operation wire 42 and plate member 44 are electrically conductive.

The operation wire 42 is formed by spirally twisting thin steel wires. The distal end of the operation wire 42 and proximal end of the plate member 44 are fixed by a first connection chip (connection part) 46a having conductivity. The first connection chip 46a connects the distal end of the operation wire 42 and the proximal end of the plate member 44 in the state that they are fixed by crimping. Therefore, the operation wire 42 and plate member 44 are electrically connected by the first connection chip 46a. When the operation wire 42 is operated, the plate member 44 is movable in the axial direction of the flexible tube 12, and rotatably about the axis.

An incision wire 18 is connected to the distal end of the plate member 44. The incision wire 18 and the distal end of the plate member 44 are fixed by a second connection chip 46b having conductivity. The second connection chip 46b connects the distal end of the plate member 44 and the proximal end of the incision wire 18 in the state that they are abutted against each other and crimped. Therefore, the plate member 44 and the incision wire 18 are electrically connected by the second connection chip 46b. When the operation wire 42 is operated, the incision wire 18 is movable together with the plate member 44 in the axial direction of the flexible tube 12, and rotatably about the axis of the flexible tube 12.

Particularly, both ends of the incision wire 18 are fixed to the distal end of the plate member 44 by the second connection chip 46b. Therefore, the incision wire 18 forms a loop 18a. The incision wire 18 is pre-stressed to open like a loop. Namely, the incision wire 18 has an elastic energizing force such that it naturally adopts a loop shape. When the incision wire 18 is pulled into the flexible tube 12 with respect to the distal end, the incision wire 18 is elastically deformed and crushed to make it thin. Namely, the incision wire 18 is formed to the state that two linear members are arranged side by side. Contrarily, when the incision wire 18 is projected with respect to the distal end of the flexible tube 12, the incision wire 18 is opened to a loop shape by the elastic energizing force.

At this time, the incision wire 18 forms the loop 18a in one plane P (refer to FIG. 4A), as shown in FIGS. 2A to 4A. Namely, the incision wire 18 is provided in the distal end portion of the plate member 44 in the state preventing a twist.

For this purpose, the loop 18a has a loop surface 18b surrounded by the incision wire 18. The loop surface 18b prevents a twist in the incision wire 18, and has directivity in a plane. The loop surface 18b is provided parallel or substantially parallel to the flat parts 44a and 44b of the plate member 44.

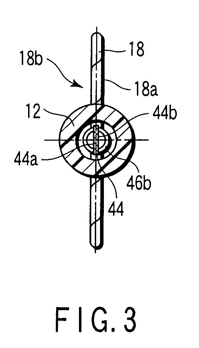

As shown in FIGS. 2B and 3, the plate member 44 is shaped like a strip. The plate member 44 has first and second flat parts 44a and 44b. The plate member 44 can be bent in a first bending direction vertical to the first and second flat parts 44a and 44b (in the direction of the normal to the first and second flat parts 44a and 44b), and in a second bending direction different from the first bending direction. When bending the plate member 44 in the second bending direction, a force larger than that required for bending in the first bending direction is required. The second bending direction is a direction vertical to the first bending direction, for example.

As shown in FIGS. 4A and 4B, the insertion section 62 of the endoscope 60 has a distal end rigid portion 62a, a bending portion 62b, and a flexible portion 62c, sequentially from the distal end side to the proximal end side. Therefore, the treatment apparatus insertion channel 64 constitutes the insertion section 62 in the state that the flexible portion 62c, bending portion 62b and distal end rigid portion 62a are inserted. At the distal end of the treatment apparatus insertion channel 64, a treatment apparatus raiser 66 is provided to bend a treatment apparatus inserted into the channel 64 (the flexible tube 12 and operation wire 42 of the high-frequency snare 10a in the embodiment) in a desired direction. The treatment apparatus raiser 66 is operated by a not-shown operation unit in the proximal end portion of the insertion section 62. The bending portion 62b of the insertion section 62 is bent in a desired direction by operating the operation unit (not shown) in the proximal end portion of the insertion section 62.

The flexible tube 12 of the high-frequency snare 10a is inserted into the treatment apparatus insertion channel 64 of the endoscope 60, the distal end of the flexible tube 12 is projected from the distal end of the channel 65, and the incision wire 18 is projected from the distal end of the flexible tube 12. At this time, the proximal end of the plate member 44 of the high-frequency snare 10a is placed in the area of the distal end rigid portion 62a of the insertion section 62. Namely, the arrangement and lengths of the flexible tube 12 and operation wire 42 of the high-frequency snare 10a are defined to have this state in relation to the treatment apparatus insertion channel 64 of the endoscope 60. Therefore, the plate member 44 of the high-frequency snare 10a is placed avoiding the bending portion 62b of the insertion section 62 of the endoscope 60, and the plate member 44 is prevented from interfering with the bending portion 62b.

Next, explanation will be given on the process of removing a polyp L in the body cavity by using the high-frequency snare 10a of this embodiment in combination with the endoscope 60.

The insertion section 62 of the endoscope 60 having the treatment apparatus raiser 66 of the channel 64 at the vicinity of the distal end of the insertion section 62 is inserted into the body cavity. The flexible tube 12 of the high-frequency snare 10a is inserted into the channel 64 of the insertion section 62. Then, the flexible tube 12 of the high-frequency snare 10a is inserted into the body cavity. At this time, the incision wire 18 of the high-frequency snare 10a is being pulled into the flexible tube 12.

The flexible tube 12 of the high-frequency snare 10a is projected with respect to the distal end of the insertion section 62. Namely, the distal end of the flexible tube 12 of the high-frequency snare 10a is projected into the body cavity. The slider 24 in the operation handle 14 is advanced to the base member 22. The operation wire 42 is moved forward to the flexible tube 12. Therefore, the incision wire 18 is moved forward through the plate member 44. As shown in FIGS. 2A and 4A, the incision wire 18 of the high-frequency snare 10a projects with respect to the distal end of the flexible tube 12. The incision wire 18 projected from the distal end of the flexible tube 12 is opened by its elastic force, and forms the loop 18a.

Next, the operation unit (not shown) of the endoscope 60 is operated, and the treatment apparatus raiser 66 provided at the distal end of the channel 64 of the insertion section 62 is raised, as shown in FIG. 4B. Then, the distal end portion of the flexible tube 12 of the high-frequency snare 10a is pressed and bent by the raiser 66.

When the distal end portion of the flexible tube 12 is bent, the plate member 44 provided inside is also bent together with the flexible tube 12. At this time, the plate member 44 is bent in the first bending direction by a force weaker than the force of bending in the second bending direction. Therefore, the plate member 44 is rotationally moved by bending in the first bending direction inside the flexible tube 12. Namely, the plate member 44 is rotationally moved about its longitudinal axis until the direction of one of the flat parts 44a and 44b of the plate member 44 coincides with the rising direction of the treatment apparatus raiser 66.

Therefore, as the plate member 44 is rotationally moved, the distal end of the operation wire 42 and the incision wire 18 are rotationally moved. Namely, as the plate member 44 is rotationally moved according to the rising operation of the treatment apparatus raiser 66, the loop surface 18b of the loop 18a of the incision wire 18 is rotated until it coincides with the rising direction of the treatment apparatus raiser 66.

Therefore, as shown in FIG. 4B, the normal line N of the plane P including the loop surface 18b of the incision wire 18 is directed to the rising direction of the polyp L. Namely, the rising direction of the polyp L crosses the loop surface 18b. Even if the loop 18a of the incision wire 18 is faced to any other direction when projecting, it can be directed to a predetermined direction with respect to the insertion section 62 of the endoscope 60 by using the raiser 66. This operation can be done while observing the loop 18a of the incision wire 18 or polyp L through the endoscope 60.

The direction of the loop 18a of the incision wire 18 can be adjusted to a direction such that the polyp L is easily caught. Therefore, the whole incision wire 18 can be directed to the polyp L. The loop 18a of the incision wire 18 can be easily and securely hung on the base (neck) of the polyp L.

In this state, the slider 24 of the high-frequency snare 10a is retracted with respect to the base member 22. Then, the incision wire 18 is pulled with respect to the distal end of the flexible tube 12. Namely, the diameter of the loop 18a is gradually decreased. Therefore, the base of the polyp L is tightened by the incision wire 18. In this state, a high-frequency current is supplied to the electrode 34 of the operation handle 14 of the high-frequency snare 10a from a high-frequency power supply. A high-frequency current then flows from the electrode 34 to the incision wire 18 through the operation wire 42 and plate member 44, and the polyp L is cut off. After cutting off the polyp L, supply of the high-frequency current is stopped.

The same operation is performed when cutting off another polyp (not shown). After cutting another polyp, the flexible tube 12 is removed from the channel 64 in the state that the incision wire 18 is being pulled into the flexible tube 12.

As explained above, the following can be said according to this embodiment.

Generally, even if the incision wire 18 is projected from the distal end of the flexible tube 12 and the loop 18a is opened, the direction of the loop surface 18b is not determined. Unless the direction of the loop surface 18b in the normal N (refer to FIGS. 4A and 4B) is faced to the polyp L, it is difficult to hang the loop 18a on the polyp L. For example, as shown in FIG. 4A, if the plane P including the loop 18a does not face the polyp L, it is difficult to hang the loop 18a on the polyp L.

In this embodiment, when the treatment apparatus raiser 66 of the endoscope 60 is raised, the distal end portion of the flexible tube 12 is bent. Then, as the raiser 66 is raised, the plate member 44 is rotated about its longitudinal axis until the flat part 44a or 44b of the plate member 44 faces in the same direction as the direction the raiser 66 is rising in. Namely, the plate member is rotated according to the rising of the treatment apparatus raiser 66, until the direction of the loop surface 18b of the loop 18a coincides with the direction the treatment apparatus raiser 66 rises in. Therefore, the normal line N of the plane P including the loop 18a is always directed to a predetermined direction, as shown in FIG. 4B.

As described above, even if the loop 18a of the incision wire 18 faces any direction when projecting, the loop 18a of the incision wire 18 can be adjusted in a direction that enables the polyp L to be easily snared. Therefore, the incision wire 18 can be easily and securely hung on the polyp L.

In this embodiment, in the state that the distal end portion of the plate member 44 is projected from the distal end of the insertion section 62 of the endoscope 60, the proximal end portion of the plate member 44 is placed in the area of the distal end rigid portion 62a of the insertion section 62 of the endoscope 60. Therefore, the plate member (direction control member) 44 to control the direction of the incision wire 18 is not placed in the area of the bending portion 62b of the proximal end portion of the distal end rigid portion 62a, and the plate member 44 has sufficient length to prevent interfering with the bending of the bending portion 62b. Therefore, when adjusting the direction of the plate member 44 by the raiser 66, the adjustment is not influenced by the bending shape of the bending portion 62. Namely, controlling the direction of the plate member 44 can be performed only by the treatment apparatus raiser 66.

The plate member 44 may be placed so that at least a part of the plate member 44 is fit in a bendable range R (refer to FIG. 4A) that the distal end of the flexible tube 12 can be bent by the treatment apparatus raiser 66. The bendable range mentioned here is located in the distal end side of the flexible tube 12, and is a set of parts bent by raising the treatment apparatus raiser 66 when the flexible tube 12 is projected by optional appropriate length from the opened portion at the distal end of the channel 64 of the endoscope 60. Adopting such a configuration, the influence of the bending shape of the insertion section 62 of the endoscope 60 upon the plate member 44 and incision wire 18 can be minimized.

In this embodiment, the proximal end portion of the plate member 44 is placed in the area of the distal end rigid portion 62a. However, the proximal end portion of the plate member 44 may be ranged in the area of the bending portion 62b unless it influences the bending of the treatment apparatus raiser 66. In this case, the plate member 44 is preferably provided with an extension 44c whose proximal end portion is made narrower or thinner than the distal end portion. In this case, the extension 44c of the plate member 44 has the same function as the operation wire 42, and when the treatment apparatus raiser 66 is raised, the influence of bending the bending portion 62b of the endoscope 60 upon the plate member 44 can be reduced to a minimum.

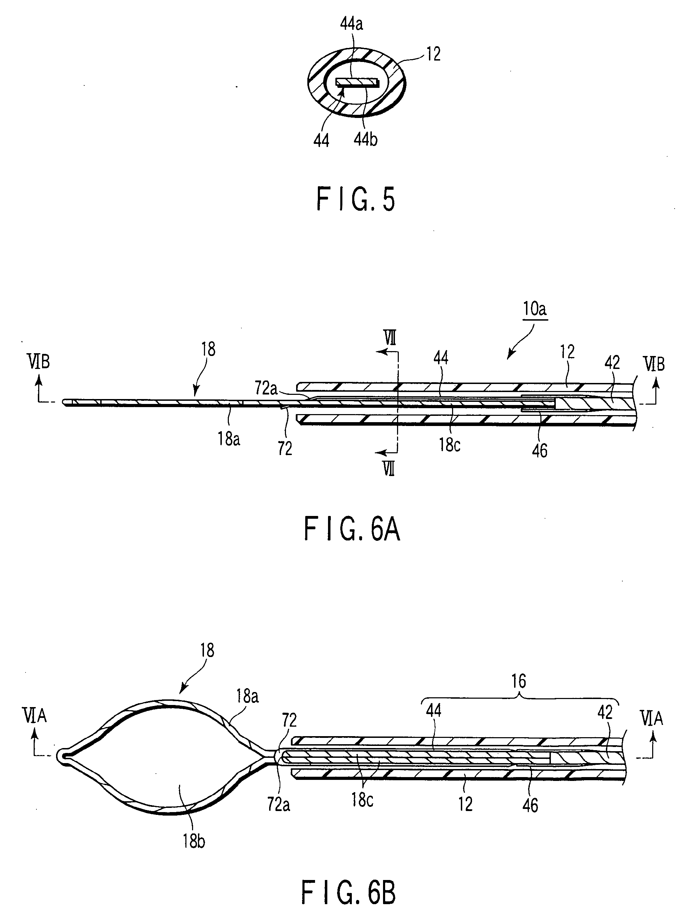

In this embodiment, the cross section of the flexible tube 12 is circular. However, as shown in FIG. 5, a cross section of the flexible tube 12 of an elliptical shape is also preferable. Namely, the flexible tube 12 may preferably be shaped flat. In this case, when bending the flexible tube 12 by the treatment apparatus raiser 66, the flexible tube 12 can be faced in a predetermined direction by rotating the flexible tube 12 itself about its axis. This can decrease the force required to raise (bend) the flexible tube 12 by the treatment apparatus raiser 66.

### (Embodiment 2)

A high-frequency incision/resection apparatus according to a second embodiment of the invention will be explained with reference to FIGS. 6A to 9. This embodiment is a modification of the first embodiment. The same reference numerals are given to the same members used in the first embodiment or the members having the same functions, and detailed explanations of these members will be omitted.

A high-frequency snare 10a according to the embodiment is configured as described below.

As shown in FIGS. 6A and 6B, the distal end of the operation wire 42 of the movable body 16 and the proximal end of the incision wire 18 are crimped and fixed by the connection chip 46. At the distal end of the connection chip 46, the proximal end of the plate member 44 is crimped and fixed together with the operation wire 42 and incision wire 18.

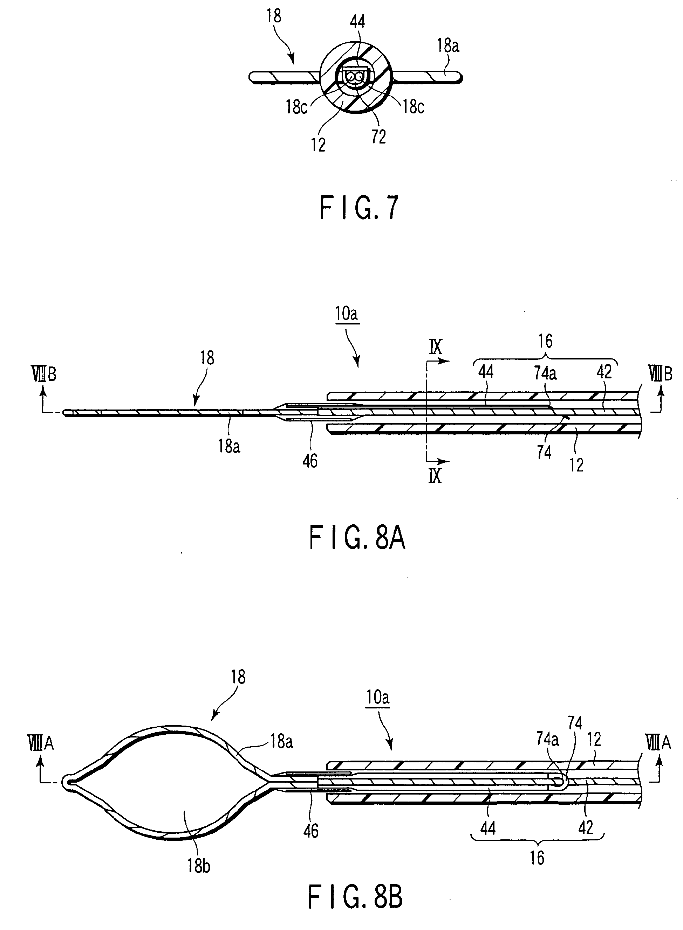

As shown in FIG. 6A, in the distal end portion of the plate member 44, a bent portion 72 slightly bent with respect to the proximal end side is formed. A through hole 72a is formed in the bent portion 72, as shown in FIGS. 6A and 7. Therefore, in FIGS. 6A and 7, the incision wire 18 is provided under the proximal end side of the plate member 44, and the incision wire 18 is extended to the distal end side through the through hole 72 of the bent portion 72 in the distal end portion of the plate member 44. Namely, the proximal end of the incision wire 18 is placed parallel to the plate surface direction of the plate member 44.

The configuration of the high-frequency snare 10a is otherwise unchanged from that of the first embodiment. The function is also the same as the first embodiment. Therefore, explanations on the configuration and function will be omitted.

As explained above, the following can be said according to this embodiment.

In this embodiment, the incision wire 18 and operation wire 42 are connected by using the connection chip 46. Compared with the first embodiment, the number of connecting points can be decreased by one. Therefore, the rigid portion can be made shorter than the rigid portion that is added by the connection chips 46a and 46b (refer to FIGS. 2A and 2B) in the first embodiment.

Further, when cutting off the polyp L, a tensile force on tightening applied to the incision wire 18 can be directly transmitted to the incision wire 18 without passing through the plate member 44. Therefore, the plate member 44 is prevented from being exposed to a large load as far as possible.

The incision wire 18 or operation wire 42 is provided parallel to the plate member 44. Therefore, the plate member 44 is prevented from plastically deforming due to exposure to a large load.

The operation wire 42 is fixed only by the proximal end of the plate member 44, but the operation wire 42 is not fixed at the other end. Namely, the proximal end of the plate member 44 is a fixing end for the operation wire 42, but the distal end of the plate member 44 is a free end for the operation wire 42. Therefore, when the plate member 44 is bent, the incision wire 18 is moved with respect to the through hole 72a of the bent portion 72 at the distal end of the plate member 44. This can prevent the plate member 44 from becoming difficult to bend in the first bending direction.

In this embodiment, the incision wire and operation wire 42 are fixed at the proximal end of the plate member 44, as shown in FIGS. 6A to 7. As a modification of a second embodiment, it is preferable to fix the incision wire 18 and operation wire 42 at the distal end of the plate member 44, as shown in FIGS. 8A to 9.

Namely, as shown in FIGS. 8A and 8B, the distal end of the operation wire 42 of the movable body 16 and the proximal end of the incision wire 18 are fixed by crimping by the connection chip 46. Further, at the proximal end of the connection chip 46, the distal end of the plate member 44 is crimped and fixed together with the operation wire 42 and incision wire 18.

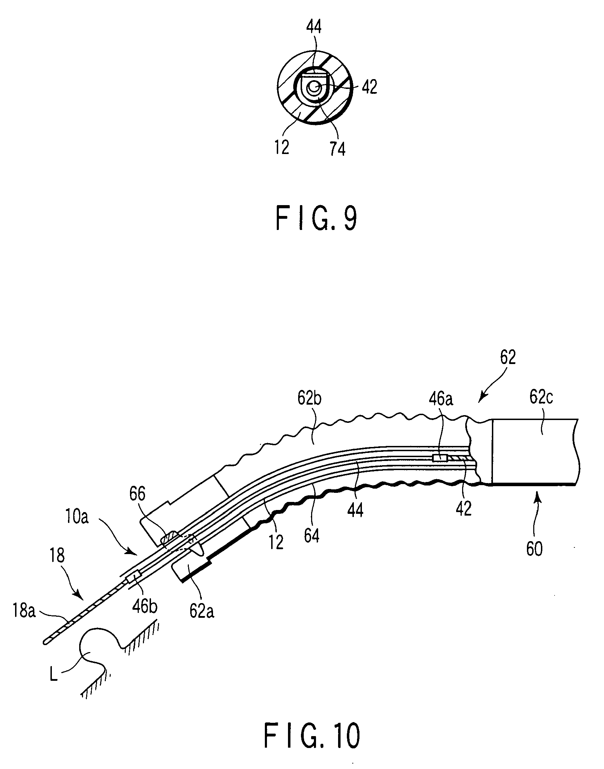

As shown in FIG. 8A, in the proximal end portion of the plate member 44, the bent portion 74 is formed slightly bent with respect to the distal end side. As shown in FIGS. 8A to 9, the through hole 74a is formed in the bent portion 74. Therefore, in FIGS. 8A and 9, the incision wire 18 is provided under the distal end side of the plate member 44. The distal end portion of the plate member 44 is fixed to through the through hole 74a of the bent portion 74 in the proximal end portion of the plate member 44. Namely, the distal end of the plate member 44 is a fixed end for the operation wire 42, but the proximal end of the plate member 44 is a free end for the operation wire 42.

In the above configuration, no rigid connection portion exists in the operation wire 42, and the operation wire is connected to the incision wire 18 in the distal end portion. Therefore, the operation wire 42 can be smoothly moved.

### (Embodiment 3)

A high-frequency incision/resection apparatus according to a third embodiment of the invention will be explained with reference to FIG. 10. This embodiment is a modification of the first embodiment. The same reference numerals are given to the same members used in the first embodiment or the members having the same functions, and detailed explanations of these members will be omitted.

FIG. 10 shows an example of using the high-frequency snare 10a as a high-frequency incision/resection apparatus.

In the high-frequency snare 10a, only the length of the plate member 44 is different from the plate member 44 in the first embodiment. Concretely, the plate member 44 of the third embodiment is made longer than the plate member 44 of the first embodiment. In the third embodiment, the flexible tube 12 of the high-frequency snare 10a is inserted into the treatment apparatus insertion channel 64 of the endoscope 60, and the distal end of the flexible tube 12 is projected from the distal end of the channel 64, and the incision wire 18 is projected from the distal end of the flexible tube 12. At this time, the proximal end portion of the plate member 44 is placed in the area of the bending portion 62b of the endoscope 60. Namely, the arrangement and lengths of the flexible tube 12 and operation wire 42 of the high-frequency snare 10a are defined to have this state in relation to the treatment apparatus insertion channel 64 of the endoscope 60.

Therefore, when bending the bending portion 62b of the insertion section 62 of the endoscope 60 in the same direction as the direction the treatment apparatus raiser 66 rises in, the rising by the treatment apparatus raiser 66 can be controlled more securely. Further, by bending the bending portion 62b of the insertion section 62 of the endoscope 60, the direction of the plate member 44 can be controlled to a certain extent without operating the treatment apparatus raiser 66.

### (Embodiment 4)

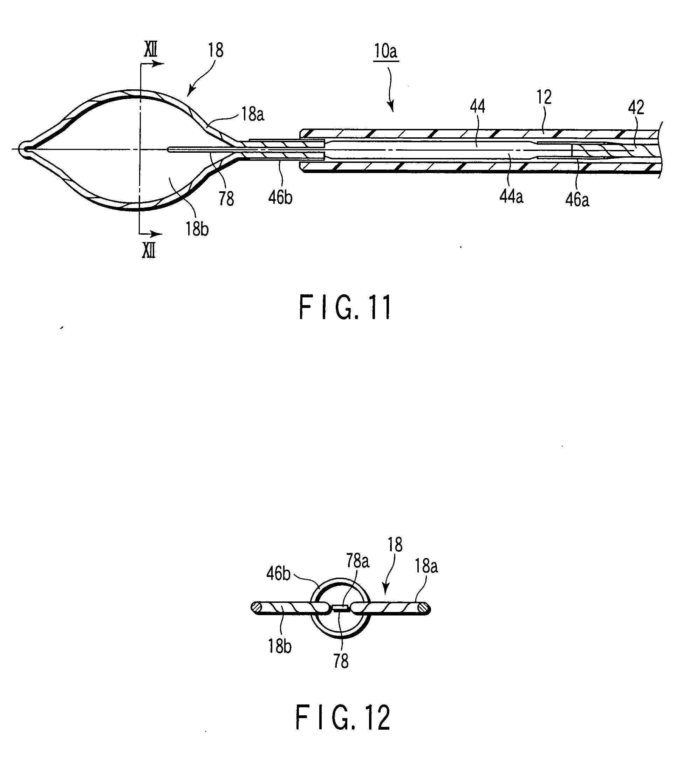

A high-frequency incision/resection apparatus according to a fourth embodiment of the invention will be explained with reference to FIGS. 11 to 13. This embodiment is a modification of the first embodiment. The same reference numerals are given to the same members used in the first embodiment or the members having the same functions, and detailed explanations of these members will be omitted.

As shown in FIG. 11, at the distal end of the plate member 44, a projection 78 is formed projecting inside the loop surface 18b, which opposes the surface of a living tissue caught in the loop 18a of the incision wire 18, extending over the second connection chip 46b. As shown in FIG. 12, the projected flat surface 78a of the projection 78 is parallel to the loop surface 18b of the loop 18a. As shown in FIG. 11, the projecting length of the projection 78 is preferably about 1/3 of the length of the loop 18a of the incision wire 18 when placed in the natural state. The projecting length of the projection 78 is the length to interfere with the treatment apparatus raiser 66, when the width of the loop 18a is largely decreased.

Next, explanation will be given on the process of cutting off the polyp L in the body cavity by using the high-frequency snare 10a of this embodiment in combination with the endoscope 60.

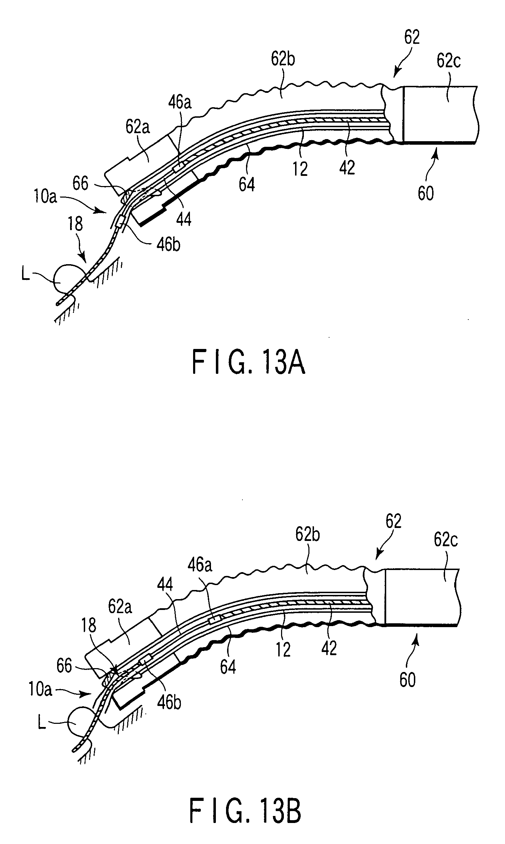

The loop 18a is hung on the polyp L, as shown in FIG. 13A. In this state, the slider 24 of the high-frequency snare 10a is retracted with respect to the base member 22. Then, the incision wire 18 is pulled to the distal end of the flexible tube 12.

In this time, even if the second connection chip 46b is pulled from the position of the treatment apparatus raiser 66 of the endoscope 60, since the projection 78 (refer to FIG. 11) is being pressed by the treatment apparatus raiser 66 the direction of the projection is controlled, as is the plate member 44. Therefore, even if the second connection chip 46b is pulled from the position of the treatment apparatus raiser 66 of the endoscope 60, the direction of loop 18a of the incision wire 18 is prevented from changing.

### (Embodiment 5)

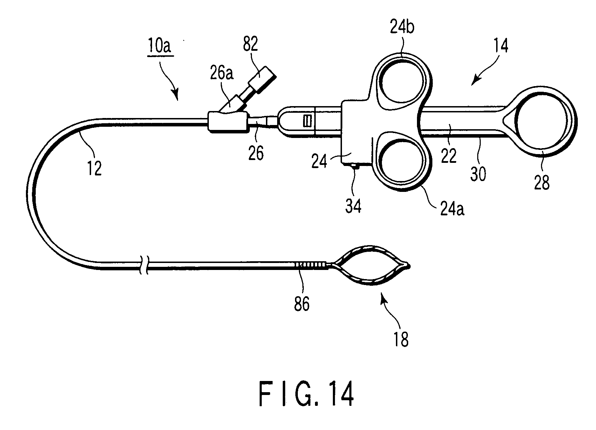

A high-frequency incision/resection apparatus according to a fifth embodiment of the invention will be explained with reference to FIGS. 14 to 16. This embodiment is a modification of the first embodiment. The same reference numerals are given to the same members used in the first embodiment or the members having the same functions, and detailed explanations of these members will be omitted.

The high-frequency snare 10a of this embodiment differs from the first embodiment in the following configuration.

As shown in FIG. 14, the proximal end portion of the flexible tube 12 of the high-frequency snare 10a of the fifth embodiment is provided with a cylindrical projection 26a at the position of the protection hood 26. In the projection 26a, a knob 82 is provided movably in a predetermined range with respect to the projection 26a. The knob 82 is fixed to the proximal end of the wire 88, described later.

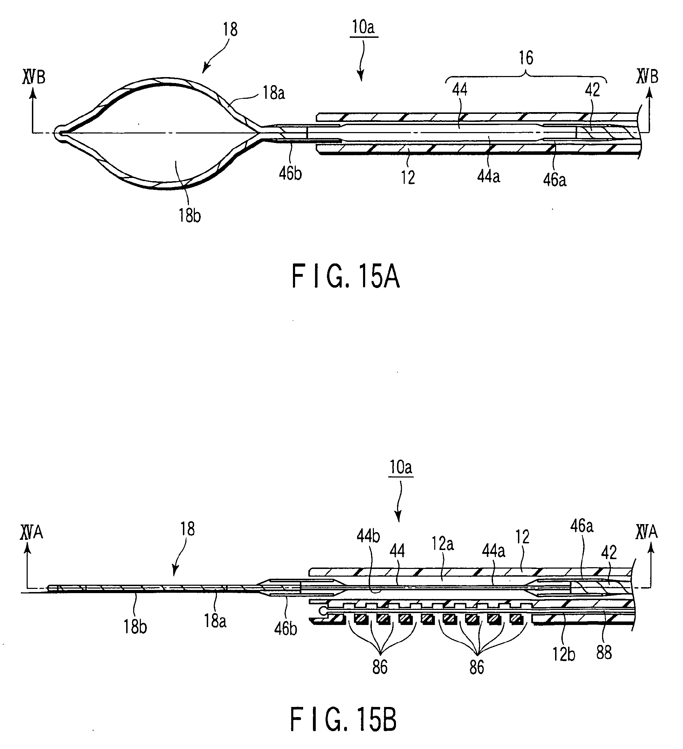

As shown in FIG. 15B, the flexible tube 12 is provided with a first inner hole 12a and a second inner hole 12b, which are eccentric over the longer axis direction. In the first inner hole 12a, the movable body 16 having the incision wire 18, plate member 44 and operation wire of the same configuration as that explained in the first embodiment is inserted.

The distal end portion of the flexible tube 12 is provided with notches 86 that penetrate the inner hole 12b. These notches 86 are formed with a predetermined interval in the longer axis direction, in the direction perpendicular to the longer axis direction of the second inner hole 12b.

One wire 88 is inserted into the second inner hole 12b. The distal end of the wire 88 is shaped like a sphere. The spherical distal end of the wire 88 is engaged or fixed at the distal end of the flexible tube 12. The wire 88 is extended to the operation handle 14 through the second inner hole 12b. The proximal end of the wire 88 is fixed to the knob 82. Therefore, when the knob 82 is operated, the wire 88 is slid inside the second inner hole 12b. In this time, movement of the distal end of the wire 88 is controlled, and when the knob 82 is slid forward, the wire 88 is loosened. Then, the distal end portion of the flexible tube 12 provided with notches 86 is bent in the direction in which the notches 86 open. Contrarily, when the knob 82 is slid rearward, the wire 88 is pulled. Therefore, the portion provided with the notches 86 is bent in the direction in which the notches 86 close.

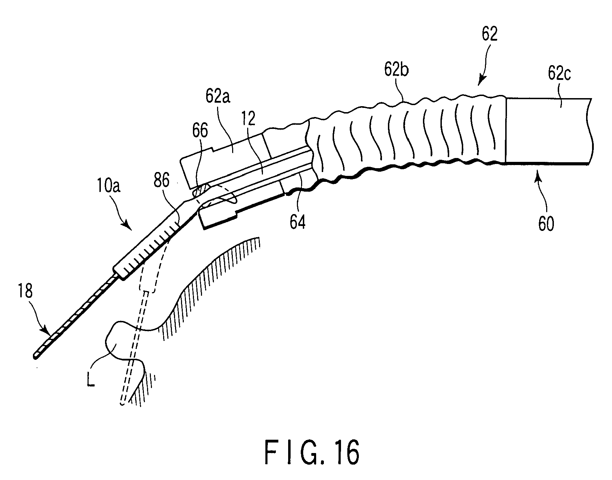

In this embodiment, such a raising-aid device (notches 86) is provided in the distal end portion of the flexible tube 12. As shown in FIG. 16, the loop 18a of the incision wire 18, whose loop surface 18b has been faced to the rising direction by the treatment apparatus raiser 66 of the endoscope 60, can be moved further in the rising direction. Therefore, the loop 18a can be hung on the polyp L more easily.

### (Embodiment 6)

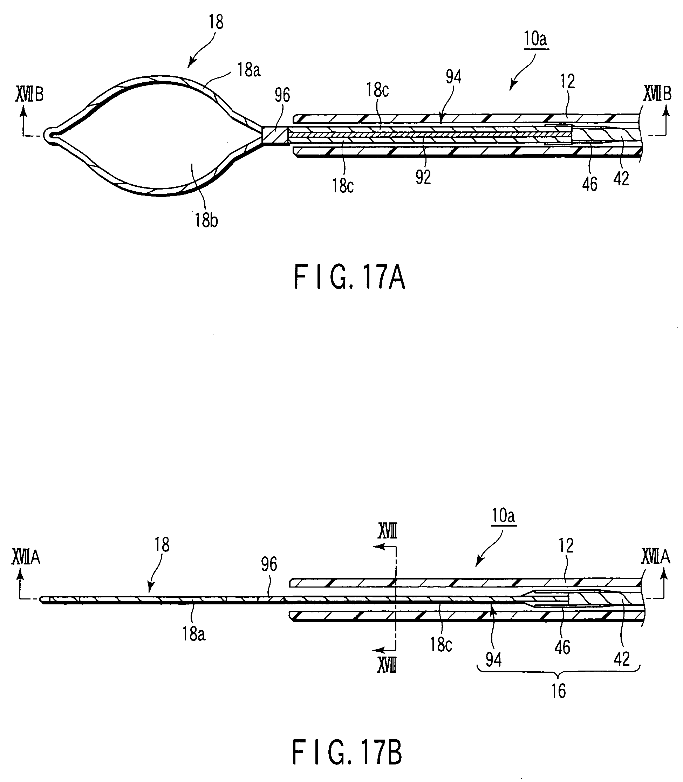

A high-frequency incision/resection apparatus according to a sixth embodiment of the invention will be explained with reference to FIGS. 17A to 19. This embodiment is a modification of the first embodiment. The same reference numerals are given to the same members used in the first embodiment or the members having the same functions, and detailed explanations of these members will be omitted.

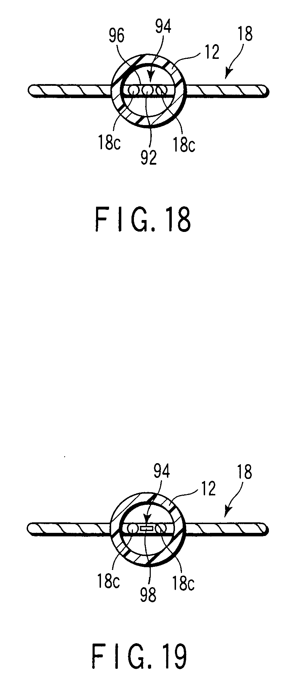

As shown in FIGS. 17A and 17B, the incision wire 18 has an extension 18c formed longer than the proximal end of the incision wire 18 explained in the first embodiment, in the proximal end portion. A thin core member 92 is provided parallel to the extension 18c of the incision wire 18. Particularly, the core member 92 is held between the extensions 18c of the incision wire 18, as shown in FIG. 18. Therefore, the proximal end portion of the incision wire 18 is formed wide by the extension 18c and core member 92. The core member 92 is made harder than the incision wire 18. Therefore, the core member 92 can be used to reinforce the proximal end portion of the incision wire 18. The core member 92 is made of a solid wire, for example. Further, the core member 92 is made to have substantially the same diameter as the incision wire 18. As described above, the core member 92 and the extension 18c of the incision wire 18 constitute a direction control member 94, which controls the direction of the incision wire 18 by rotationally moving about its longitudinal axis. Namely, the direction control member 94 controls the direction of the incision wire 18 by rotationally moving about its longitudinal axis, by being pressed by the treatment apparatus raiser 66, like the plate member 44 explained in the first embodiment.

The extension 18c of the incision wire 18 and the distal end of the core member 92 shown in FIG. 17A are fixed by a fixing material 96, for example, by brazing. The proximal end of the extension 18c of the incision wire 18 and the proximal end of the core member 92 are fixed to the distal end of the operation wire 42 by the connection chip 46, as shown in FIG. 17A.

The configuration is otherwise the same as the first embodiment. The function is also the same as the first embodiment.

As configured above, the following can be said about this embodiment, in addition to the explanation in the first embodiment.

Compared with the first embodiment, the loop 18a is not provided with the second connection chip 46b, and a height difference is not generated between the direction control member 94 and loop 18a. Therefore, when projecting and pulling the loop 18a with respect to the distal end of the flexible tube 12, the loop 18a can be prevented from catching on the treatment apparatus raiser 66 of the endoscope 60. This makes treatment smooth.

Further, as shown in FIG. 19, the direction control member 94 may be composed of a narrow plate member 98 instead of the core member 92 (refer to FIGS. 17A and 18). In this case, the plate member 98 is thinner in thickness and substantially the same in width, compared with the diameter of the extension 18c of the incision wire 18.

### (Embodiment 7)

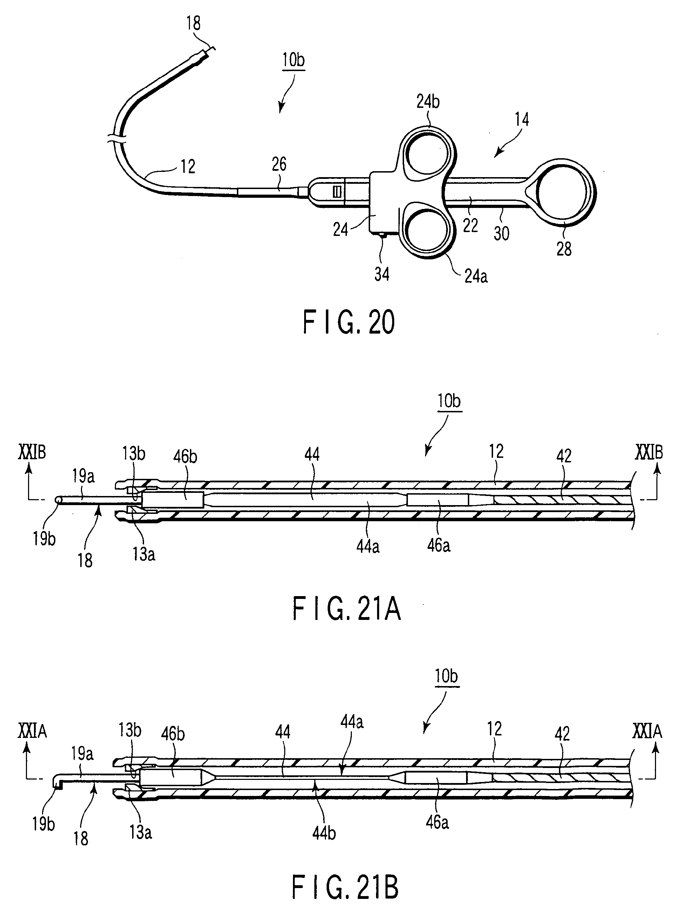

A high-frequency incision/resection apparatus according to a seventh embodiment of the invention will be explained with reference to FIGS. 20 to 23. This embodiment is a modification of the first embodiment. The same reference numerals are given to the same members used in the first embodiment or the members having the same functions, and detailed explanations of these members will be omitted.

In this embodiment, a high-frequency knife 10b is used as a high-frequency incision/resection apparatus.

The high-frequency knife 10b has a flexible tube 12, an operation handle 14, and a treatment portion 18. In this embodiment, the treatment portion 18 has an L-shaped electrode.

As shown in FIGS. 21A and 21B, in the high-frequency knife 10b of this embodiment, an L-shaped electrode 18 is connected to the distal end of the plate member 44 through the second connection chip 46b. A stopper 13a having a venthole 13b is fixed to the inside surface of the distal end portion of the flexible tube 12. The venthole 13b has a diameter sufficient to permit movement of a linear part 19a described later of the L-shaped electrode 18, but insufficient to permit providing a bent portion 19b inside the flexible tube 12.

As shown in FIGS. 21A and 21B, the L-shaped electrode 18 includes the linear part 19a disposed on the central axis of the operation wire 42 and a bent portion 19b formed at its distal end by bending rectangularly to the linear part 19a. The linear part 19a is movable in the venthole 13b of the stopper 13a. The bent portion 19b is extended in the direction vertical to the flat parts 44a and 44b of the plate member 44. Namely, the bent portion 19b is bent rectangularly to the linear part 19a, and the diameter of the venthole 13b is made smaller than the length of the bent portion 19a. Therefore, the bent portion 19b cannot pass through the venthole 13b of the stopper 13a.

The second connection chip 46b is formed larger than the venthole 13b. The second connection chip 46b is provided inside the flexible tube 12, and cannot pass through the venthole 13b. Therefore, the second connection chip 46b contacts the stopper 13a, and the projecting length of the L-shaped electrode 18 with respect to the distal end of the flexible tube 12 is controlled. The L-shaped electrode 18 can be moved with respect to the venthole 13b by the length equivalent to the distance from the bent portion 19b to the distal end of the connection chip 46b.

The configuration is otherwise the same as the first embodiment.

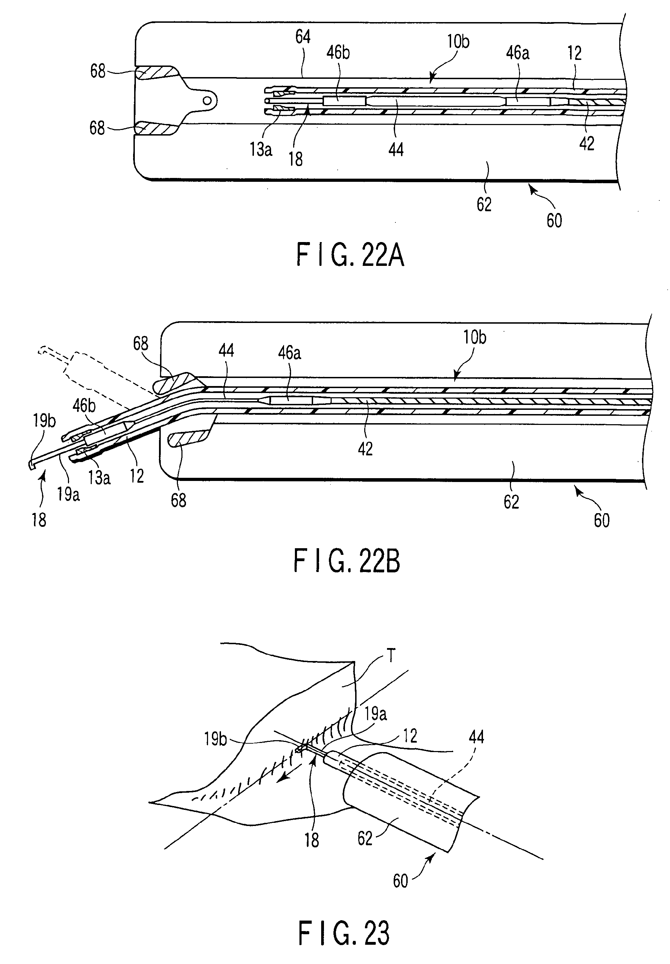

As shown in FIGS. 22A and 22B, a cylindrical swing rising pad 68 is provided at the distal end of the channel 64 of the endoscope 60. The swing rising pad 68 can swing to the left and right, and rise in any direction.

Next, explanation will be given on the process of cutting off a tissue layer T under a mucous membrane (refer to FIG. 23) in the body cavity by using the high-frequency knife 10b of this embodiment in combination with the endoscope 60.

The insertion section 62 of the endoscope 60 having the swing rising pad 68 is inserted in the proximity of the channel 64. The flexible tube 12 of the high-frequency knife 10b is inserted into the channel 64 of the insertion section 62. At this time, the flexible tube 12 of the high-frequency knife 10b is projected with respect to the distal end of the insertion section 62 of the endoscope 60 through the treatment rising pad 68. Then, the flexible tube 12 of the high-frequency knife 10b is inserted into the body cavity. In this time, the L-shaped electrode 18 is being pulled inside the flexible tube 12.

The flexible tube 12 of the high-frequency knife 10b is projected with respect to the distal end of the insertion section 62. Namely, the distal end of the flexible tube 12 of the high-frequency knife 10b is projected into the body cavity. The slider 24 in the operation handle 14 is then advanced to the base member 22. Therefore, the operation wire 42 is moved forward with respect to the flexible tube 12. Then, the L-shaped electrode 18 is moved forward through the plate member 44. At this time, the L-shaped electrode 18 of the high-frequency knife 10b projects to the distal end of the flexible tube 12, and the second connection chip 46b butts against the stopper 13a.

When projecting the L-shaped electrode 18 from the distal end of the flexible tube 12, the bent portion 19b of the L-shaped electrode 18 may not be faced in a direction for cutting the tissue layer T. In this case, the distal end portion of the flexible tube 12 is raised by the swing rising pad 68. Then, as shown in FIG. 22B, the plate member 44 located at the proximal end of the L-shaped electrode 18 is rotationally moved in the flexible tube 12 so that the flat parts 44a and 44b coincide with the rising direction of the switch rising pad 68. Therefore, the L-shaped electrode 18 is also rotationally moved so that the bent portion 19b coincides with the rising direction of the treatment apparatus raiser 66. At this time, the insertion section 62 is moved rotationally about its axis, and the bent portion 19b is faced in the rising direction, that is, the direction in which the tissue layer T under a mucous membrane is cut. Therefore, the L-shaped electrode 18 can be easily operated.

As shown in FIG. 23, the bent portion 19b of the L-shaped electrode 18 is hung on the tissue layer T under a mucous membrane. The L-shaped electrode 18 is moved to the tissue layer T while flowing a high frequency current to the L-shaped electrode 18 from the electrode 34 of the slider 24 of the operation handle 14. Then, the tissue layer T is cut and removed.

### Industrial Applicability

According to the present invention, there can be provided an endoscopic treatment apparatus and an endoscope system which can face a treatment portion in a desired direction in the body cavity regardless of how the endoscope is bent by controlling the direction of a direction control member.

## Claims

1. An endoscopic treatment device (10a, 10b) which is configured to be used with an endoscope (60) including an insertion section (62) having a treatment device raiser (66, 68), and a treatment device insertion channel (64) provided along the insertion section (62), and which is insertable into the insertion channel (64) of the endoscope (60), the endoscopic treatment device (10a, 10b) comprising:
a flexible tube (12) including a distal end portion, a proximal end portion and a lumen connecting the distal end portion and the proximal end portion, and the flexible tube (12) being configured to be inserted into the treatment device insertion channel (64);
a treatment portion (18) configured to project and retract from the distal end portion of the flexible tube (12), the treatment portion (18) being rotatable about the axial direction of the flexible tube (12);
an operation handle (14) disposed on the proximal end of the flexible tube (12); and
a movable body (16) which includes a distal end connected to the treatment portion (18), and a proximal end connected to the operation handle (14) wherein the movable body (16) is inserted into the lumen of the flexible tube (12) and is configured to move along the axial direction and to move the treatment portion (18) along the axial direction in accordance with the movement of the operation handle (14),
the movable body (16) comprising an operation wire (42) and a direction control member (44) connected to the distal end of the operation wire (42), the direction control member (44) configured to be bent in a first direction perpendicular to the axial direction, and in a second direction perpendicular to the first direction and the axial direction, with a force greater than in the first direction being required for bending in the second direction,
wherein:
when the flexible tube (12) is bent by the treatment device raiser (66, 68), the direction control member (44) is rotationally moved about its longitudinal axis until the first direction of the direction control member (44) coincides with the bending direction of the flexible tube (12) such that the treatment portion (18) is in a predetermined orientation with respect to the insertion section (62) of the endoscope which predetermined orientation is defined by the bending direction of the direction control member (44) and the flexible tube (12)
**characterised in that**
the orientation of the direction control member (44) with respect to the axial direction is independent from any rotational position of the flexible tube (12) relative to the insertion section (62) of the endoscope (60).

2. The endoscopic treatment device (10a, 10b) according to claim 1, wherein the flexible tube (12) and the direction control member (44) are bendable according to the activation of the treatment device raiser (66) when the flexible tube (12) is projected from a distal end of the insertion section (62) and the treatment portion (18) is projected from a distal end of the flexible tube (12).

3. The endoscopic treatment device (10a, 10b) according to claim 1, wherein the treatment portion (18) includes an opposite surface (18b) with a normal direction in the same direction as the first direction of the direction control member (44), and the opposite surface (18b) is configured to be faced to a living tissue (L, T).

4. The endoscopic treatment device (10a, 10b) according to claim 1, wherein the direction control member (44) is formed of a plate member, the first direction of the direction control member (44) is a thickness direction of the plate member, the second direction of the direction control member (44) is a width direction of the plate member, and the width direction of the direction control member (44) is longer than the thickness direction.

5. The endoscope treatment device (10a, 10b) according to claim 1, wherein the operation member (42) is formed of an operation wire (42) at which the direction control member (44) is disposed at a distal end side thereof.

6. The endoscope treatment device (10a, 10b) according to claim 5, wherein the treatment portion (18) is connected to a distal end of the operation wire (42), the operation handle (14) is connected to a proximal end of the operation wire (42), and the direction control member (44) is arranged beside the operation wire (42) at a distal end side of the operation wire (42).

7. The endoscopic treatment device (10a, 10b) according to claim 1, wherein the direction control member (94) includes:
one core member (92); and
a wire member which is aligned on both sides of the core member (92), and has a hardness less than the core member (92).

8. The endoscopic treatment device (10a, 10b) according to claim 1, wherein the treatment portion (18) includes a snare with a loop (18a), and the direction control member (44) includes a flat part (44a, 44b) configured to be parallel to a loop surface (18b) formed by the loop (18a).

9. The endoscopic treatment device (10a, 10b) according to claim 1, wherein the direction control member (44) includes:
a distal end portion fixed to a distal end of the operation member (42), and to a proximal end of the treatment portion (18); and
a proximal end having a bent portion (74) with a through hole (74a) configured to slidably receive the operation member (42).

10. An endoscope system comprising:
an endoscopic treatment device (10a, 10b) according to claim 1, and
an endoscope (60) including:
a thin elongated insertion section (62);
a channel (64) which is configured for insertion of the flexible tube (12) of the endoscopic treatment device (10a, 10b) therein; and
a treatment device raiser (66, 68) configured to bend the flexible tube (12), the operation member (42), and the direction control member (44) of the endoscopic treatment device (10a, 10b), to a rising direction.

## Patentansprüche

1. Endoskopische Behandlungsvorrichtung (10a, 10b), welche dazu eingerichtet ist, mit einem Endoskop (60) verwendet zu werden, das einen Einführungsabschnitt (62) umfasst, der einen Behandlungsvorrichtungsheber (66, 68) und einen entlang des Einführungsabschnitts (62) vorgesehenen Behandlungsvorrichtungseinführungskanal (64) aufweist, und welche in den Einführungskanal (64) des Endoskops (60) einführbar ist, wobei die endoskopische Behandlungsvorrichtung (10a, 10b) umfasst:
ein flexibles Rohr (12) umfassend einen distalen Endabschnitt, einen proximalen Endabschnitt und ein Lumen, welches den distalen Endabschnitt und den proximalen Endabschnitt verbindet, und wobei das flexible Rohr (12) dazu eingerichtet ist, in den Behandlungsvorrichtungseinführungskanal (64) eingeführt zu werden;
einen Behandlungsabschnitt (18), welcher dazu eingerichtet ist, aus dem distalen Endabschnitt des flexiblen Rohrs (12) herauszuragen und sich darin zurückzuziehen, wobei der Behandlungsabschnitt (18) um die axiale Richtung des flexiblen Rohrs (12) drehbar ist;
einen Operationsgriff (14), welcher an dem proximalen Ende des flexiblen Rohrs (12) angeordnet ist; und
einen beweglichen Körper (16), welcher ein distales Ende, welches mit dem Behandlungsabschnitt (18) verbunden ist, und ein proximales Ende, welches mit dem Operationsgriff (14) verbunden ist, umfasst, wobei der bewegliche Körper (16) in das Lumen des flexiblen Rohrs (12) eingeführt ist und dazu eingerichtet ist, sich entlang der axialen Richtung zu bewegen und den Behandlungsabschnitt (18) entlang der axialen Richtung gemäß der Bewegung des Operationsgriffs (14) zu bewegen,
wobei der bewegliche Körper (16) einen Operationsdraht (42) und einen mit dem distalen Ende des Operationsdrahts (42) verbundenen Richtungssteuerungsabschnitt (44) aufweist, wobei der Richtungssteuerungsabschnitt (44) dazu eingerichtet ist, in eine erste Richtung senkrecht zu der axialen Richtung und in eine zweite Richtung senkrecht zu der ersten Richtung und der axialen Richtung gebogen zu werden, wobei zum Biegen in die zweite Richtung eine größere Kraft als in die erste Richtung erforderlich ist,
wobei:
wenn das flexible Rohr (12) von dem Behandlungsvorrichtungsheber (66, 68) gebogen wird, der Richtungssteuerungsabschnitt (44) drehbar um seine longitudinale Achse bewegt wird, bis die erste Richtung des Richtungssteuerungsabschnitts (44) mit der Verbiegungsrichtung des flexiblen Rohrs (12) übereinstimmt, so dass sich der Behandlungsabschnitt (18) in einer vorbestimmten Ausrichtung in Bezug auf den Einführungsabschnitt (62) des Endoskops befindet, wobei die vorbestimmte Ausrichtung durch die Verbiegungsrichtung des Richtungssteuerungsabschnitts (44) und des flexiblen Rohrs (12) definiert wird,
**dadurch gekennzeichnet, dass**
die Ausrichtung des Richtungssteuerungsabschnitts (44) in Bezug auf die axiale Richtung unabhängig von jeglicher Drehposition des flexiblen Rohrs (12) relativ zum Einführungsabschnitt (62) des Endoskops (60) ist.

2. Endoskopische Behandlungsvorrichtung (10a, 10b) gemäß Anspruch 1, wobei das flexible Rohr (12) und der Richtungssteuerungsabschnitt (44) biegbar entsprechend der Aktivierung des Behandlungsvorrichtungshebers (66) sind, wenn das flexible Rohr (12) aus einem distalen Ende des Einführungsabschnitts (62) herausragt und der Behandlungsabschnitt (18) aus dem distalen Ende des flexiblen Rohrs (12) herausragt.

3. Endoskopische Behandlungsvorrichtung (10a, 10b) gemäß Anspruch 1, wobei der Behandlungsabschnitt (18) eine gegenüberliegende Fläche (18b) mit einer Normalenrichtung in dieselbe Richtung wie die erste Richtung des Richtungssteuerungsabschnitts (44) aufweist und die gegenüberliegende Fläche (18b) dazu eingerichtet ist, einem lebenden Gewebe (L, T) gegenüberzustehen.

4. Endoskopische Behandlungsvorrichtung (10a, 10b) gemäß Anspruch 1, wobei der Richtungssteuerungsabschnitt (44) aus einem Plattenabschnitt gebildet ist, wobei die erste Richtung des Richtungssteuerungsabschnitts (44) eine Dickenrichtung des Plattenabschnitts ist, die zweite Richtung des Richtungssteuerungsabschnitts (44) eine Breitenrichtung des Plattenabschnitts ist und die Breitenrichtung des Richtungssteuerungsabschnitts (44) länger ist als die Dickenrichtung.

5. Endoskopische Behandlungsvorrichtung (10a, 10b) gemäß Anspruch 1, wobei der Operationsabschnitt (42) von einem Operationsdraht (42) gebildet ist, an welchem der Richtungssteuerungsabschnitt (44) an einer distalen Endseite davon angebracht ist.

6. Endoskopische Behandlungsvorrichtung (10a, 10b) gemäß Anspruch 5, wobei der Behandlungsabschnitt (18) mit einem distalen Ende des Operationsdrahtes (42) verbunden ist, der Operationsgriff (14) mit einem proximalen Ende des Operationsdrahtes (42) verbunden ist und der Richtungssteuerungsabschnitt (44) neben dem Operationsdraht (42) an einer distalen Endseite des Operationsdrahtes (42) angeordnet ist.

7. Endoskopische Behandlungsvorrichtung (10a, 10b) gemäß Anspruch 1, wobei der Richtungssteuerungsabschnitt (94) umfasst:
einen Kernabschnitt (92); und
einen Drahtabschnitt, welcher an beiden Seiten des Kernabschnitts (92) ausgerichtet ist und eine geringere Härte als der Kernabschnitt (92) aufweist.

8. Endoskopische Behandlungsvorrichtung (10a, 10b) gemäß Anspruch 1, wobei der Behandlungsabschnitt (18) eine Schlinge mit einer Schlaufe (18a) umfasst und
der Richtungssteuerungsabschnitt (44) einen flachen Abschnitt (44a, 44b) umfasst, der dazu eingerichtet ist, parallel zu einer von der Schlaufe (18a) gebildete Schlaufenfläche (18b) zu sein.

9. Endoskopische Behandlungsvorrichtung (10a, 10b) gemäß Anspruch 1, wobei der Richtungssteuerungsabschnitt (44) umfasst:
einen an einem distalen Ende des Operationsabschnitts (42) und an einem proximalen Ende des Behandlungsabschnitts (18) befestigten distalen Endabschnitt; und
ein proximales Ende, welches einen verbogenen Abschnitt (74) mit einem Durchgangsloch (74a) aufweist, eingerichtet zum einschiebbaren Aufnehmen des Operationsabschnitts (42).

10. Endoskopsystem umfassend:
eine endoskopische Behandlungsvorrichtung (10a, 10b) gemäß Anspruch 1 und
ein Endoskop (60) umfassend:
einen dünnen länglichen Einführungsabschnitt (62);
einen Kanal (64), welcher eingerichtet ist zum darin Einführen des flexiblen Rohrs (12) der endoskopischen Behandlungsvorrichtung (10a, 10b); und
einen Behandlungsvorrichtungsheber (66, 68), eingerichtet zum Biegen des flexiblen Rohrs (12), des Operationsabschnitts (42) und des Richtungssteuerungsabschnitts (44) der endoskopischen Behandlungsvorrichtung (10a, 10b) in eine Heberichtung.

## Revendications

1. Dispositif (10a, 10b) de traitement endoscopique qui est configuré pour être utilisé avec un endoscope (60) incluant une section d'insertion (62) comportant un élément de soulèvement (66, 68) de dispositif de traitement, et un canal d'insertion (64) de dispositif de traitement prévu le long de la section d'insertion (62), et qui peut être inséré dans le canal d'insertion (64) de l'endoscope (60), le dispositif (10a, 10b) de traitement endoscopique comprenant :
un tube flexible (12) incluant une partie d'extrémité distale, une partie d'extrémité proximale et une lumière connectant la partie d'extrémité distale et la partie d'extrémité proximale, et le tube flexible (12) étant configuré pour être inséré dans le canal d'insertion (64) de dispositif de traitement ;
une partie de traitement (18) configurée pour faire saillie et se rétracter par rapport à la partie d'extrémité distale du tube flexible (12), la partie de traitement (18) étant rotative autour de la direction axiale du tube flexible (12) ;
une poignée d'actionnement (14) disposée sur l'extrémité proximale du tube flexible (12) ; et
un corps mobile (16) qui comprend une extrémité distale connectée à la partie de traitement (18), et une extrémité proximale connectée à la poignée d'actionnement (14), dans lequel le corps mobile (16) est inséré dans la lumière du tube flexible (12) et est configuré pour se déplacer le long de la direction axiale et pour déplacer la partie de traitement (18) le long de la direction axiale conformément au mouvement de la poignée d'actionnement (14),
le corps mobile (16) comprenant un câble d'actionnement (42) et un élément de commande de direction (44) connecté à l'extrémité distale du câble d'actionnement (42), l'élément de commande de direction (44) étant configuré pour être courbé dans une première direction perpendiculaire à la direction axiale, et dans une deuxième direction perpendiculaire à la première direction et à la direction axiale, une force supérieure à celle dans la première direction étant nécessaire pour être courbé dans la deuxième direction,
dans lequel :
lorsque le tube flexible (12) est courbé par l'élément de soulèvement (66, 68) de dispositif de traitement, l'élément de commande de direction (44) est déplacé en rotation autour de son axe longitudinal jusqu'à ce que la première direction de l'élément de commande de direction (44) coïncide avec la direction de courbure du tube flexible (12) d'une manière telle que la partie de traitement (18) se trouve dans une orientation prédéterminée par rapport à la section d'insertion (62) de l'endoscope dont l'orientation prédéterminée est définie par la direction de courbure de l'élément de commande de direction (44) et du tube flexible (12)
**caractérisé en ce que**
l'orientation de l'élément de commande de direction (44) par rapport à la direction axiale est indépendante de n'importe quelle position rotationnelle du tube flexible (12) par rapport à la section d'insertion (62) de l'endoscope (60).

2. Dispositif (10a, 10b) de traitement endoscopique selon la revendication 1, dans lequel le tube flexible (12) et l'élément de commande de direction (44) peuvent être courbés conformément à l'activation de l'élément de soulèvement (66) de dispositif de traitement lorsque le tube flexible (12) fait saillie depuis une extrémité distale de la section d'insertion (62) et que la partie de traitement (18) fait saillie depuis une extrémité distale du tube flexible (12).

3. Dispositif (10a, 10b) de traitement endoscopique selon la revendication 1, dans lequel la partie de traitement (18) comprend une surface opposée (18b) avec une direction normale dans la même direction que la première direction de l'élément de commande de direction (44), et la surface opposée (18b) est configurée pour être en regard d'un tissu vivant (L, T).

4. Dispositif (10a, 10b) de traitement endoscopique selon la revendication 1, dans lequel l'élément de commande de direction (44) est formé d'un élément de plaque, la première direction de l'élément de commande de direction (44) correspond à une direction d'épaisseur de l'élément de plaque, la deuxième direction de l'élément de commande de direction (44) correspond à une direction de largeur de l'élément de plaque, et la direction de largeur de l'élément de commande de direction (44) est plus longue que la direction d'épaisseur.

5. Dispositif (10a, 10b) de traitement endoscopique selon la revendication 1, dans lequel l'élément d'actionnement (42) est formé d'un câble d'actionnement (42) au niveau duquel l'élément de commande de direction (44) est disposé au niveau d'un côté d'extrémité distale de celui-ci.

6. Dispositif (10a, 10b) de traitement endoscopique selon la revendication 5, dans lequel la partie de traitement (18) est connectée à une extrémité distale du câble d'actionnement (42), la poignée d'actionnement (14) est connectée à une extrémité proximale du câble d'actionnement (42), l'élément de commande de direction (44) est agencé à côté du câble d'actionnement (42) au niveau d'un côté d'extrémité distale du câble d'actionnement (42).

7. Dispositif (10a, 10b) de traitement endoscopique selon la revendication 1, dans lequel l'élément de commande de direction (44) comprend :
un élément central (92) ; et
un élément de câble qui est aligné sur les deux côtés de l'élément central (92), et présente une rigidité inférieure à l'élément central (92).

8. Dispositif (10a, 10b) de traitement endoscopique selon la revendication 1, dans lequel la partie de traitement (18) comprend une anse avec une boucle (18a), et
l'élément de commande de direction (44) comprend une partie plate (44a, 44b) configurée pour être parallèle à une surface de boucle (18b) formée par la boucle (18a).

9. Dispositif (10a, 10b) de traitement endoscopique selon la revendication 1, dans lequel l'élément de commande de direction (44) comprend :
une partie d'extrémité distale fixée à une extrémité distale de l'élément d'actionnement (42), et à une extrémité proximale de la partie de traitement (18) ; et
une extrémité proximale comportant une partie de courbure (74) avec un trou traversant (74a) configuré pour recevoir de manière coulissante l'élément d'actionnement (42).

10. Système d'endoscope comprenant :
un dispositif (10a, 10b) de traitement endoscopique selon la revendication 1, et
un endoscope (60) comprenant :
une section d'insertion (62) allongée mince ;
un canal (64) qui est configuré pour l'insertion du tube flexible (12) du dispositif (10a, 10b) de traitement endoscopique dans celui-ci ; et
un élément de soulèvement (66, 68) de dispositif de traitement configuré pour courber le tube flexible (12), l'élément d'actionnement (42), et l'élément de commande de direction (44) du dispositif (10a, 10b) de traitement endoscopique, vers une direction de soulèvement.
